# DEMANDE DE BREVET EUROPEEN

(11) **EP 1 953 538 A1**
(43) Date de publication de la demande: **06.08.2008**
(21) Numéro de dépôt: 08001187.7
(22) Date de dépôt: 23.01.2008
(51) Int. Cl.: G01N 27/06, G01N 27/22, G01N 33/28

(54) **Dispositif et procédé permettant de déterminer la dilution en liquide de refroidissement d'une huile de moteur thermique**

(30) Priorité: 30.01.2007 FR 0700606
(71) Demandeur: SC2N, 94000 Créteil (FR)
(72) Inventeur: Witemberg, Vincent, 14123 IFS (FR)
(74) Mandataire: Rolland, Jean-Christophe

(57) **Abrégé**

La présente invention a pour objet un dispositif et un procédé permettant de déterminer la dilution en liquide de refroidissement d'une huile de moteur thermique, caractérisé par le fait qu'il comprend les étapes suivantes :
- mesurer la résistance électrique (R0) et la capacité électrique (C0) d'une huile à évaluer,
- comparer le couple de valeurs (R0, C0) ainsi mesuré à une zone (Z) de couples de valeurs de résistance électrique (R) et de capacité électrique (C) située au-delà d'une courbe de référence (CR) correspondant à l'évolution de la résistance (R) en fonction de la capacité (C) pour une huile sans dilution de liquide de refroidissement.

## Description

La présente invention concerne le domaine du contrôle de la qualité de l'huile d'un moteur thermique utilisé sur véhicule automobile.

Plus précisément, la présente invention concerne la détermination de la dilution par liquide de refroidissement dans l'huile moteur.

De nombreux processus de contrôle de la qualité d'une huile moteur ont déjà été proposés. Cependant, aucun des processus connus ne donne totalement satisfaction.

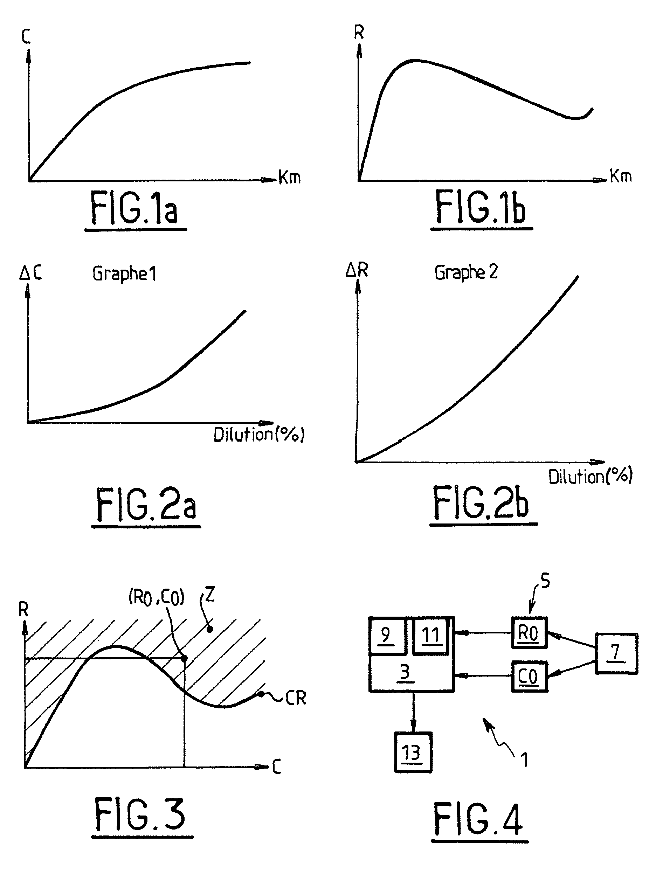

Il est bien connu en particulier que la valeur de la capacité électrique et de la résistance électrique d'une huile, varient en fonction de son utilisation, notamment dans le cas d'un véhicule diesel. On a représenté sur les figures 1a et 1b, des courbes typiques d'évolution de la valeur de la capacité électrique et de la résistance électrique de l'huile en fonction d'un kilométrage parcouru.

Sur cette base, une méthode classique pour déterminer une limite de l'état de l'huile pour sa vidange est de fixer une limite seuil sur la valeur de la capacité électrique qui augmente progressivement avec le vieillissement de l'huile.

Cette méthode est efficace dans le cas où l'huile est à vidanger à cause de son vieillissement classique. La mesure de la capacité qui sert de paramètre déterminant pour imposer la vidange prend en effet en compte entre autre, l'augmentation de la viscosité, l'état d'oxydation et la teneur en matière charbonneuse résultant de l'usure de l'huile.

En revanche, lorsqu'une dilution en liquide de refroidissement apparaît dans l'huile, la méthode précitée ne fonctionne pas. En effet, la résistance électrique de l'huile et la capacité électrique de l'huile varient simultanément avec la dilution en liquide de refroidissement.

On connaît déjà du document FR2803038, un dispositif de mesure de l'usure d'huile sur véhicule automobile mesurant la phase de l'impédance du lubrifiant.

Dans un mode de réalisation du document, les moyens de mesure du dispositif comprennent des moyens de comparaison de l'amplitude de la phase de l'impédance du lubrifiant avec une valeur seuil (S1) et des moyens aptes à générer une alerte "de vidange ou maintenance" quand le seuil précité (S1) est franchi par défaut. Le seuil (S1) peut être considéré comme un seuil relatif basé sur la mesure de la valeur initiale de la phase de l'impédance.

Pour cela, le dispositif comprend des moyens aptes à appliquer une tension alternative entre deux électrodes plongées dans un échantillon de lubrifiant à surveiller, et des moyens aptes à mesurer la phase du courant correspondant par rapport à ladite tension appliquée.

Cependant, lorsque l'utilisateur ajoute un complément d'huile neuve dans l'huile à mesurer, la phase de l'huile diminue, y compris dans le cas où l'huile est polluée en liquide de refroidissement. Il n'est alors plus possible de déterminer la pollution dans l'huile.

La présente invention a pour but de proposer une nouvelle méthode permettant de déterminer la dilution en liquide de refroidissement dans une huile de moteur thermique.

Ce but est atteint dans le cadre de la présente invention, grâce à un procédé comprenant les étapes suivantes :
- mesurer la résistance électrique et la capacité électrique d'une huile à évaluer,
- comparer le couple de valeurs (R0, C0) ainsi mesuré à une zone (Z) de couples de valeurs de résistance électrique (R) et de capacité électrique (C) située au-delà d'une courbe de référence (CR) correspondant à l'évolution de la résistance (R) en fonction de la capacité (C) pour une huile sans dilution de liquide de refroidissement.

Avantageusement, le procédé comporte en outre une étape de génération d'alerte lorsque le couple de valeurs mesuré appartient à cette zone.

La présente invention concerne également un dispositif pour la mise en oeuvre du procédé précité.

D'autres caractéristiques, buts et avantages de la présente invention apparaîtront à la lecture de la description détaillée qui va suivre et en regard des dessins annexés, donnés à titre d'exemple non limitatif et sur lesquels:
- les figures 1a et 1b, précédemment décrites, représentent deux courbes connues illustrant l'évolution de paramètres d'huile,
- les figures 2a et 2b, représentent deux courbes illustrant l'évolution de paramètres d'huile en cas de pollution de l'huile en liquide de refroidissement,
- la figure 3 représente une courbe de référence pour une usure normale, ou gabarit, délimitant une zone de valeurs pour une huile polluée en liquide de refroidissement,
- la figure 4 représente une vue schématique d'un dispositif de contrôle de la qualité de l'huile, conforme à la présente invention.

Comme on l'a évoqué précédemment, dans le cas d'un usage normal, le vieillissement d'une huile conduit à l'augmentation progressive de la capacité électrique (voir figure 1a) et à une augmentation rapide suivie d'une diminution progressive de la résistance électrique (voir figure 1b); en fonction des kilomètres parcourus.

D'autre part, les composantes résistive et capacitive de l'huile varient en fonction des éléments susceptibles de provenir d'une pollution par un liquide de refroidissement.

Les essais réalisés par la demanderesse, schématisés sur les figures 2a et 2b annexées, ont révélé qu'en cas de pollution de l'huile par un corps étranger tel qu'un liquide de refroidissement, résultant par exemple d'une défaillance mécanique, les résistances électriques et la capacité électrique de d'huile augmentent.

Le déposant a en outre constaté que les courbes de la résistance électrique et de la capacité électrique, augmentent toutes deux de façon monotone avec l'augmentation de la dilution.

Plus précisément encore, le déposant a constaté que, plus la dilution augmente et plus la résistance et la capacité électrique du lubrifiant s'éloignent des valeurs d'usure normale de l'huile sans dilution.

La présente invention permet de déterminer la dilution en liquide de refroidissement d'une huile de moteur thermique en déterminant si l'évolution des paramètres de l'huile correspond à une usure normale ou à une dilution.

Pour cela, le processus de détermination de la dilution en liquide de refroidissement comprend une première étape de mesure de la résistance électrique R0 et la capacité électrique C0 de l'huile à analyser, à un instant donné.

Puis, dans une seconde étape, le couple de valeurs R0, C0 ainsi mesuré est comparé à une zone Z de couples de valeurs de résistance électrique R et de capacité électrique C située au-delà d'une courbe de référence CR comme illustré sur la figure 3.

La courbe de référence ou gabarit, correspond à l'évolution R = F (C) de la résistance R en fonction de la capacité électrique C d'une huile sans dilution en liquide de refroidissement correspondant à un vieillissement classique.

La zone Z est située dans la partie située au-delà de la courbe de référence CR et comprend toutes les valeurs de couple R, C pour lesquels, à une valeur donnée de capacité C, la valeur de la résistance R correspondante est supérieure à la valeur de la résistance R obtenue par la courbe de référence CR pour la même valeur de capacité C.

Etant donné qu'en cas de pollution, la résistance R et la capacité C électrique de l'huile augmentent simultanément, tous les couples de valeurs R0, C0 appartenant à la zone Z correspondent à une huile diluée en liquide de refroidissement.

La mesure de la résistance et de la capacité indépendamment l'une de l'autre, permet la différenciation entre une pollution en liquide de refroidissement et un ajout d'huile neuve. En effet, en cas d'ajout d'huile neuve, les valeurs R, C du couple mesuré ne lui permettent pas de franchir la courbe de référence CR. Ainsi, les couples R, C mesurés en cas d'ajout d'huile neuve, n'appartiennent pas à la zone Z.

Un autre avantage de la présente invention est que le procédé permet de donner une réponse sur la qualité de l'huile pour un seul couple de valeurs R, C mesuré. Le procédé ne nécessite pas la mémorisation de plusieurs valeurs successives de mesure de l'huile pour déterminer l'éventuelle pollution de celle-ci par le liquide de refroidissement.

La dilution en liquide de refroidissement du moteur présage une défaillance possible du moteur telle qu'une rupture de joint, comme le joint de culasse par exemple qui nécessite rapidement une maintenance.

Selon une caractéristique avantageuse de l'invention, le procédé comporte en outre une étape de génération d'alerte lorsque le couple de valeurs R0, C0 mesuré appartient à la zone Z pour signaler à l'utilisateur la nécessité d'intervenir sur le moteur de son véhicule.

En pratique, un dispositif 1 permet de déterminer la dilution en liquide de refroidissement d'une huile de moteur électrique conforme à la présente invention.

La figure 4 représente schématiquement le dispositif 1 qui comprend une unité de traitement 3 et des moyens de mesure 5 permettant de mesurer la résistance électrique R0 et la capacité électrique C0 d'une huile à évaluer 7.

De préférence, les moyens de mesure 5 permettent la mesure de la résistance par la mesure de la résistivité de l'huile en tension continue tandis que la capacité est mesurée à partir de la mesure de la permittivité à haute fréquence

L'unité de traitement 3, telle qu'un microprocesseur, comporte une mémoire 9 pour stocker une courbe de référence CR correspondant à l'évolution de la résistance R en fonction de la capacité électrique C pour une huile sans dilution de liquide de refroidissement.

L'unité de traitement 3 comporte en outre des moyens de comparaison 11 permettant de comparer le couple de valeurs R0, C0 ainsi mesuré à une zone Z de couples de valeurs de résistance électrique R et de capacité électrique C située au-delà d'une courbe de la courbe de référence CR en mémoire.

Selon une autre caractéristique avantageuse de l'invention, le dispositif comporte en outre des moyens d'alerte 13 pour générer une alerte lorsque le couple de valeurs R0, C0 mesuré appartient à ladite zone Z.

Pour déterminer si le couple de valeur (R0, C0) est situé ou non dans la zone Z, la comparaison du couple de valeurs (R0, C0) à la zone Z peut être effectuée comme suit.

La valeur C0 mesurée est tout d'abord reportée en abscisse d'un graphique CR (graphiquement ou numériquement grâce aux moyens de calculs des moyens de comparaison 11). Cette valeur de capacité C0 en abscisse correspond à une valeur de résistance en ordonnée donnée par la courbe de référence CR qui est connue donc par l'unité de traitement qui dispose en mémoire de cette courbe de référence. La valeur R0 mesurée est ensuite comparée à ladite valeur de résistance en ordonnée. Si la valeur R0 est égale ou inférieure, le point de coordonnées (R0, C0) est en dehors de la zone Z (pas de dilution en liquide de refroidissement de l'huile moteur). Si au contraire, elle est supérieure, le point de coordonnées (R0, C0) est dans la zone Z (voir figure 3) et une alerte peut alors être générée pour signaler la dilution en liquide de refroidissement de l'huile moteur. Le dispositif permet ainsi de mesurer la résistance et la capacité indépendamment l'une de l'autre ce qui permet, contrairement au dispositif de l'art antérieur, d'éviter les erreurs d'interprétation et de faciliter la mesure.

On comprend donc que le procédé et le dispositif de mesure de l'usure d'huile sur véhicule automobile selon l'invention résout les problèmes de l'art antérieur permettant de déterminer instantanément une pollution en liquide de refroidissement d'une usure normale ou d'un ajout de complément d'huile neuve.

## Revendications

1. Procédé permettant de déterminer la dilution en liquide de refroidissement d'une huile de moteur thermique, **caractérisé par le fait qu'**il comprend les étapes suivantes :
- mesurer la résistance électrique (R0) et la capacité électrique (CO) d'une huile à évaluer,
- comparer le couple de valeurs (R0, C0) ainsi mesuré à une zone (Z) de couples de valeurs de résistance électrique (R) et de capacité électrique (C) située au-delà d'une courbe de référence (CR) correspondant à l'évolution de la résistance (R) en fonction de la capacité (C) pour une huile sans dilution de liquide de refroidissement.

2. Procédé selon la revendication 1, **caractérisé en ce qu'**il comporte en outre une étape de génération d'alerte lorsque le couple de valeurs (R0, C0) mesuré appartient à ladite zone (Z).

3. Dispositif (1) permettant de déterminer la dilution en liquide de refroidissement d'une huile de moteur électrique, **caractérisé par le fait qu'**il comprend :
- des moyens de mesure (5) permettant de mesurer la résistance électrique (R0) et la capacité électrique (CO) d'une d'huile (7) à évaluer,
- une unité de traitement (3) comportant une mémoire (9) pour stocker une courbe de référence (CR) correspondant à l'évolution de la résistance (R) en fonction de la capacité électrique (C) pour une huile sans dilution de liquide de refroidissement,
- l'unité de traitement (3) comporte en outre des moyens de comparaison (11) permettant de comparer le couple de valeurs (R0, C0) mesuré à une zone (Z) de couples de valeurs de résistance électrique (R) et de capacité électrique (C) située au-delà de la courbe de référence (CR) en mémoire.

4. Dispositif selon la revendication 3, **caractérisé en ce qu'**il comporte en outre des moyens d'alerte (13) pour générer une alerte lorsque le couple de valeurs (R0, C0) mesuré appartient à ladite zone (Z).
